# EUROPEAN PATENT SPECIFICATION

(11) **EP 0 315 637 B1**
(45) Date of publication and mention of the grant of the patent: **06.03.1996**
(21) Application number: 87905117.5
(22) Date of filing: 22.07.1987
(51) Int. Cl.: C12P 21/00, C12N 1/20, C12N 7/00, A61K 39/00

(54) **POLYPEPTIDES USEFUL IN DIAGNOSIS OF MYCOPLASMA INFECTIONS IN SWINE AND RECOMBINANT-DNA METHODS FOR MANUFACTURING SAME**
POLYPEPTIDE ZUR VERWENDUNG BEI DER DIAGNOSE VON MYCOPLASMAINFEKTIONEN BEI SCHWEINEN, SOWIE REKOMBINANT-DNS-VERFAHREN ZUR HERSTELLUNG DERSELBEN
POLYPEPTIDES UTILES DANS LE DIAGNOSTIC D'INFECTIONS DU MYCOPLASMA CHEZ LES PORCS, ET PROCEDES D'ADN RECOMBINANT POUR LEUR FABRICATION

(30) Priority: 25.07.1986 US 889153
(43) Date of publication of application: 17.05.1989
(73) Proprietor: SYNERGEN, INC., Boulder Colorado 80301 (US)
(72) Inventor: KUNER, Jerry, M., Boulder, CO 80302 (US)
(74) Representative: Grünecker, Kinkeldey, Stockmair & Schwanhäusser Anwaltssozietät
(86) International application number: US8701785
(87) International publication number: WO8800977

(56) References cited:
- EP-A- 0 196 215
- WO-A-86/00019
- PROCEEDINGS OF THE NATL. ACADEMY OF SCIENCES USA, vol. 82, May 1985; R. YOUNG et al., pp. 2583-2587/
- INFECTION & IMMUNITY, vol. 53, no. 1, July 1986, American Society for Microbiology, Washington, DC (US); L.B. TREVINO et al., pp. 129-134/
- INFECTION & IMMUNITY, vol. 35, no. 3, March 1982, American Society for Microbiology, Washington, DC (US); D.C. KRAUSE et al., pp. 809-817/
- INFECTION & IMMUNITY, vol. 41, no. 1, July 1983, American Society for Microbiology, Washington, DC (US); S.J. GEARY et al., pp. 132-136/
- PROCEEDINGS OF THE NATL. ACADEMY OF SCIENCES USA, vol. 80, July 1983, Washington, DC (US); M.A. TAYLOR et al., pp. 4154-4158/
- INFECTION & IMMUNITY, vol. 43, January 1984, American Society for Microbiology, Washington, DC (US); F.C. MINION et al., pp. 119-120/
- INFECTION & IMMUNITY, vol. 49, no. 2, August 1985, American Society for Microbiology, Washington, DC (US); M.Q. KLINKERT et al., pp. 329-335/

## Description

### BACKGROUND OF THE INVENTION

The present invention relates to a class of polypeptides useful in diagnostic assays to determine the presence of antibodies to mycoplasma organisms in mammals, particularly in pigs or hogs. The invention also relates to recombinant-DNA methods for the manufacture of these polypeptides and recombinant phage clones containing DNA sequences suitable for use in the recombinant methods.

Enzootic pneumonia of pigs, also known as virus pneumonia, infectious pneumonia, anterior lobe pneumonia, enzootic virus pneumonia and mycoplasmal pneumonia of swine, rarely causes death, but often results in severe morbidity and reduced performance in weight gain of swine. Originally believed to be caused by a virus, it was determined in 1965 that the causative agent was Mycoplasma hyopneumoniae, also known as Mycoplasma suipneumoniae.

The disease is transmitted from pig to pig through the nasal passages by airborne organisms expelled from infected pigs. The mycoplasma establish themselves deep in the apical and cardiac lobes of the lungs where they cause visible plum colored or gray lesions and cause difficulty in breathing and reduced weight gain. The primary infection by M. hyopneumoniae may be followed by secondary infection by other mycoplasma species (M. hyorhinus and M. floculare) as well as bacterial pathogens (Pasteurella and Bordetella species).

The mycoplasmas are prokaryotic cells smaller and simpler in structure than bacteria, but more complex than viruses. Unlike viruses, they are capable of a free living existence, though they are often found in association with eukaryotic cells. They are bounded by a cell membrane but not by a cell wall. They have an extremely small genome, approximately 750,000 base pairs in length.

While this disease is not often fatal, it causes decreased growth and weight gain in the affected animals at a time when the animals are being fed for market. Thus, animals which have been infected with this organism will be worth less at slaughter than will their non-infected counterparts.

Due to the serious economic consequences of pig pneumonia, diagnostic testing methods have been sought which will indicate the presence of an infection caused by Mycoplasma hyopneumoniae in swine.

EP-A-0196215 describes surface antigens of Mycoplasma hyopneumoniae. Antisera detect in western blots of M. hyopneumoniae total cell proteins, several proteins having molecular weights of 90,000, 68,000, 50,000, 30,000 and 26,000 respectively. The present inventors have discovered a class of polypeptides useful in the diagnosis of this and certain other Mycoplasma infections. These polypeptides, when used in in vitro diagnostic assays, indicate the presence of antibodies against certain Mycoplasma organisms in infected pig and hog sera.

To facilitate use of these polypeptides, the present invention also relates to recombinant-DNA methods for manufacturing the polypeptides. These recombinant-DNA methods utilize DNA sequences contained in various recombinant phage clones which are described herein.

### SUMMARY OF THE INVENTION

It is an object of the present invention to provide polypeptides useful in the diagnosis of certain mycoplasma infections particularly Mycoplasma hyopneumoniae infections in swine. It also an object of the present invention to identify recombinant-DNA methods for the manufacture of these polypeptides.

Additional objects and advantages of the present invention will be set forth in part in the description which follows, or may be learned from the practice of the invention. The objects and advantages may be realized and attained by means of the instrumentalities and combinations particularly pointed out in the appended claims.

To achieve the objects and in accordance with the purposes of the present invention, Proteins A, C, D and E as specified in claim 1 have been disclosed. The DNA corresponding to portions of these proteins is contained on various lambda phages which also are identified herein and specified in claims 2 to 5.

In addition, a recombinant-DNA method for the manufacture of the polypeptides as specified in claim 1 is disclosed.

These proteins are capable of creating an immuno-diagnostic complex when exposed to sera from swine infected with Mycoplasma hyopneumoniae and certain other mycoplasma organisms. This method comprises:
(a) preparation of DNA sequence capable of directing a host micro-organism to produce the protein;
(b) cloning the DNA sequence into a vector capable of being transferred into and replicating in a host micro-organism, such vector containing operational elements for the DNA sequence;
(c) transferring the vector containing the DNA sequence and operational elements into a host micro-organism capable of expressing the antigenic protein;
(d) culturing the host micro-organism under conditions appropriate for amplification of the vector and expression of the protein; and
(e) in either order:
   i) harvesting the protein; and
   ii) causing the protein to assume a structure whereby it possesses antigenic properties analogous to properties possessed by Mycoplasma organisms.

### DETAILED DESCRIPTION OF THE PREFERRED EMBODIMENTS

Reference will now be made in detail to the presently preferred embodiments of the invention, which, together with the drawings and the following examples, serve to explain the principles of the invention.

As noted above, the present invention relates to a class of polypeptides which are useful, inter alia, for in vitro diagnosis of mycoplasma infection in swine. These substantially purified proteins are analogous to various Mycoplasma hyopneumaniae proteins which are believed to be capable of inducing an immune response when present in swine tissue. Because an immune response has been mounted in infected swine against analogous antigens, the sera of such infected swine will contain antibodies which will recognize one or more of the polypeptides of the present invention. Thus, the instant polypeptides may serve, either in combination or individually, as the active ingredient in an in vitro diagnostic assay to determine the presence in swine sera of antibodies directed toward various Mycoplasma species.

As used herein, the term "analogous," when used in connection with a protein, antigen or polypeptide, is intended to mean a polypeptide which is capable of detecting antibodies raised in response to an infection with natural Mycoplasma proteins in swine. A polypeptide possessing analogous antigenic properties will thus exhibit some homology to the native Mycoplasma protein. It should be noted that "analogous" polypeptides, as the term is used herein, may raise an immune response which is stronger than, the same as, or weaker than the response raised by natural Mycoplasma proteins.

The following surface proteins have been discovered by the present inventors as useful in such in vitro diagnostics. These include: Protein A, a 105kd protein of M. hyopneumoniae;

Protein C, an 85kd protein of M. hyopneumoniae; Protein D, a /0kd protein of M. hyopneumoniae; Protein E, a 43kd protein of M. hyopneumoniae. It should be noted that the molecular weights associated with the proteins disclosed herein are not to be interpreted as absolute values and were measured by SDS - PAGE. It is believed that each of the proteins A through E is a protein present on the surface of the Mycoplasma organism. When intact Mycoplasma cells are lightly treated with a protease (trypsin), each of these proteins exhibits sensitivity to digestion by the protease, indicating their exposure on the cell surface.

Moreover, it is believed that each of these proteins contain one or more specific portions which may serve as an antigenic determinant capable of binding to at least one antibody present in sera of Mycoplasma infected swine. These specific antigenic portions, either singly or in various combinations, would be, therefore, capable of serving as the basis for an in vitro diagnostic assay.

DNA sequences encoding portions of these proteins are contained on the lambda gt11 clones identified herein. The DNA sequences coding for the entire proteins are contained in the same gt11 library from which the above clones were derived, and methods are described below that will allow the identification and isolation of such clones.

A portion of the gene encoding polypeptide A (105Kd) is contained on the lambda gt11 clone R60b which contains an insert of mycoplasma DNA of 1.5 kilobases. This fragment can be excised using the restriction endonucleases Kpnl and Sacl which cut in the flanking vector sequences but not within the insert. The corresponding expression plasmid R60b-a has been constructed by insertion of the Kpnl/Sacl insertion fragment of the lambda gt11 clone into the plasmid vector pSEV6.

A portion of the gene encoding polypeptide C (85Kd) is contained on the lambda gt11 clone R69 which contains an insert of mycoplasma DNA of 0.5 kilobases. This fragment can be excised using the restriction endonucleases Kpnl and Sacl which cut in the flanking vecator sequences but not within the insert. The corresponding expression plasmid R69b has been constructed by insertion of the Kpnl/Sacl insertion fragment of the lambda gt11 clone into the plasmid vector pSEV6.

A portion of the gene encoding polypeptide D (70Kd) is contained on the lambda gt11 clone 86-4 which contains an insert of mycoplasma DNA of 3.2 kilobases. This fragement can be excised using the restriction endonucleases Kpnl and SauI which cut in the flanking vector sequences but not within the insert. The corresponding expression plasmid 86-4C has been constructed by insertion of the Kpnl/SauI insertion fragment of the lambda gt11 clone into the plasmid vector pSEV6.

A portion of the gene encoding polypeptide E (43Kd) is contained on the lambda gt11 clone P1 which contains an insert of mycoplasma DNA of 0.5 kilobases. This fragment can be excised using the restriction endonucleases Kpnl and Sacl which cut in the flanking vector sequences but not within the insert. The corresponding expression plasmid P1C has been constructed by insertion of the Kpnl/Sacl insertion fragment of the lambda gt11 clone into the plasmid vector pSEV6.

Various methods may be used to express the DNA encoding the proteins or the proposed antigenic determinants. In particular, it is contemplated that the DNA contained on the gt11 phage clones may be expressed in mammalian systems.

In an alternate preferred embodiment, it is contemplated that the DNA of interest will be excised from the DNA contained on the gt11 phage clone and inserted, in a suitable form, into a microbial expression system. In this embodiment, the antigenic polypeptides are produced by a method comprising:
(a) preparation of a DNA sequence capable of directing a host microorganism to produce a polypeptide possessing antigenic properties analogous to those possessed by a polypeptide produced by Mycoplasma organisms;
(b) cloning the DNA sequence into a vector capable of being transferred into and replicating in a host microorganism, such vector containing operational elements for the DNA sequence;
(c) transferring the vector containing the DNA sequence and operational elements into a host microorganism capable of expressing the antigenic polypeptides;
(d) culturing the host microorganism under conditions appropriate for amplification of the vector and expression of the polypeptide; and
(e) in either order:
   (i) harvesting the polypeptide; and
   (ii) causing the polypeptide to assume a structure whereby it possesses antigenic properties analogous to properties possessed by polypeptides produced by Mycoplasma organisms.

Since M. hyopneumoniae is a prokaryote, genomic DNA may be used directly without concern about introns. However, at least one mycoplasma species has been shown to utilize the normal stop codon UGA as a tryptophan codon in protein synthesis. If this is also true in M. hyopneumoniae, expression in other systems (e.g., E. coli) would result in premature termination during protein synthesis when this codon is read as a stop. Whether this occurs for the proteins of interest can be determined by growing the expression vector in suitable tRNA suppressor strains. If premature termination does occur, it will be possible to correct the problem by DNA sequencing the area containing the UGA codon and substituting the proper codon by site-directed mutagenesis. These procedures are readily accomplished by one of ordinary skill in the art.

The DNA prepared in accordance with the above methods is inserted into an expression vector suitable for use in the intended expression system. Embodiments of the present invention are envisioned as employing other known or currently undiscovered vectors which would contain one or more of the DNA sequences encoding antigenic polypeptides described herein. In particular, it is preferred that these vectors have some or all of the following characteristics: (1) possess a minimal number of host-organism sequences; (2) be stable in the desired host; (3) be capable of being present in a high copy number in the desired host; (4) possess a regulatable promoter; (5) have at least one DNA sequence coding a selectable trait present on a portion of the plasmid separate from that where DNA sequence encoding for the antigenic polypeptide will be inserted; and (6) be integrated into the vector.

The following, noninclusive, list of cloning vectors is believed to set forth vectors which can easily be altered to meet the above-criteria and are, therefore, preferred for use in the present invention. Such alterations are easily performed by those of ordinary skill in the art in light of the available literature and the teachings herein.

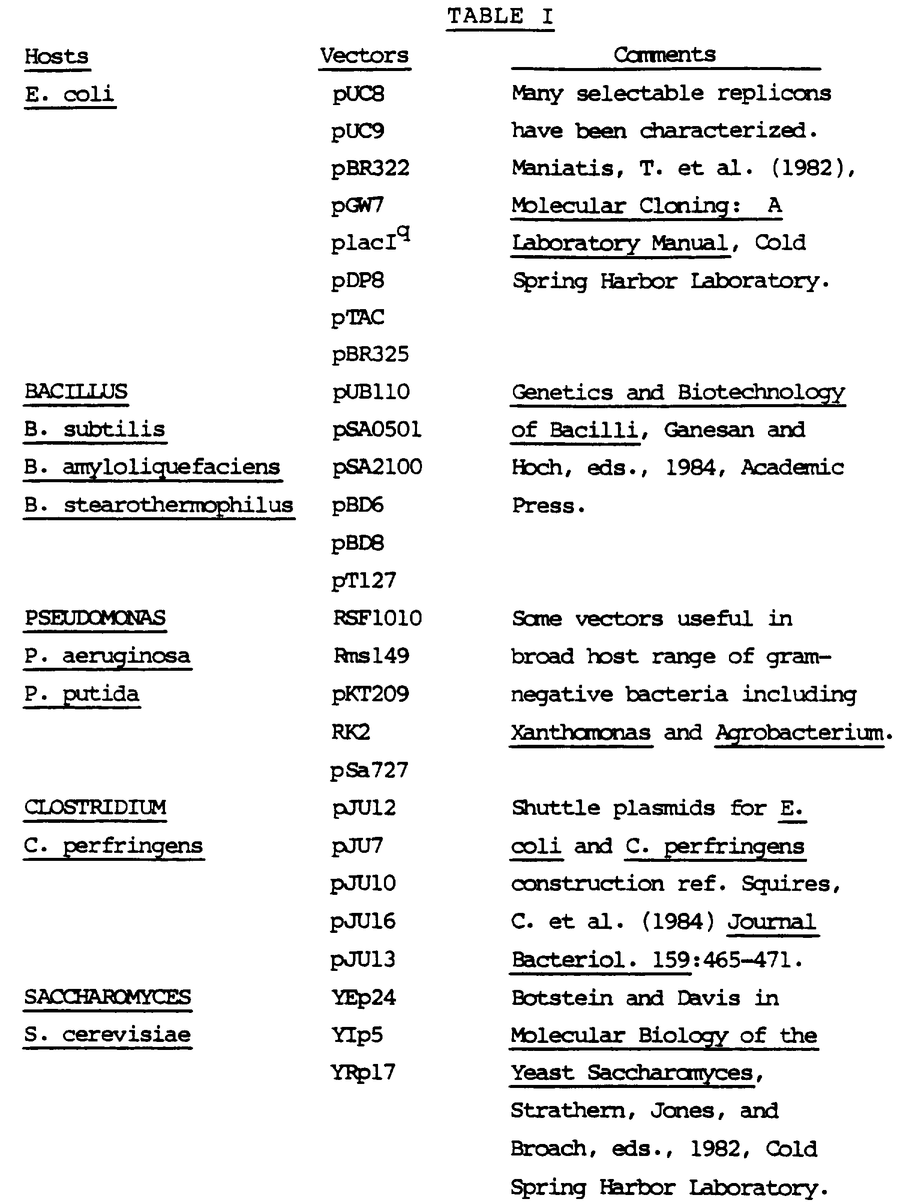

It is to be understood that additional cloning vectors may now exist or will be discovered which have the above-identified properties and are therefore suitable for use in the present invention. These vectors also are contemplated as being within the scope of the disclosed series of cloning vectors into which the DNA sequences encoding the antigenic polypeptides may be introduced, along with any necessary operational elements, and which altered vector is then included within the scope of the present invention and would be capable of being used in the recombinant-DNA method set forth more fully below.

These "operational elements," as discussed herein, include but are not limited to at least one promoter, at least one ribosome-binding sequence and at least one transcription terminator. Preferably, these "operational elements" also include at least one operator, at least one leader sequence for proteins to be exported from the intracellular space, at least one regulator and any other DNA sequences necessary or preferred for appropriate transcription and subsequent translation of the vector DNA.

In addition to the above list, an E. coli vector system is preferred in one embodiment as a cloning vector. Moreover, several vector plasmids which autonomously replicate in a broad range of Gram negative bacteria are preferred for use as cloning vehicles in hosts of the genera Pseudomonas. These are described by Tait, R.C., Close, T.J., Lundquist, R.C., Hagiya, M., Rodriguez, R.L., and Kado, C.I. in Biotechnology, May, 1983, pp. 269-275; Panopoulos, N.J. in Genetic Engineering in the Plant Sciences, Praeger Publishers, New York, New York, pp. 163-185, (1981); and Sakaguchi, K. in Current Topic in Microbiology and Immunology 96:31-45, (1982), each of which is specifically incorporated herein by reference.

One particularly preferred construction employs the plasmid RSF1010 and derivatives thereof as described by Bagdasarian, M., Bagdasarian, M.M., Coleman, S., and Timmis, K.N. in Plasmids of Medical, Environmental and Commercial Importance, Timmis, K.N. and Puhler, A., eds., Elsevier, North Holland Biomedical Press, (1979), specifically incorporated herein by reference. The advantages of RSF1010 are that it is relatively small, high copy number plasmid which is readily transformed into and stably maintained in both E. coli and Pseudomonas species. In this system, it is preferred to use the Tac expression system as described for Escherichia, since it appears that the E. coli trp promoter is readily recognized by Pseudomonas RNA polymerase as set forth by Sakaguchi, K. in Current Topics in Microbiology and Immunology 96:31-45 (1982) and Gray, G.L., McKeown, K.A., Jones, A.J.S., Seeburg, P.H., and Heyneker, H.L. in Bio/Technology, Feb. 1984, pp. 161-165, both of which are specifically incorporated herein by reference. Transcriptional activity may be further maximized by requiring the exchange of the promoter with, e.g., an E. coli or P. aeruginosa trp promoter.

In a preferred embodiment, P. aeruginosa is transformed with vectors directing the synthesis of the antigenic polypeptides as either an intracellular product or as a product coupled to leader sequences that will effect its processing and export from the cell. In this embodiment, these leader sequences are preferably selected from the group consisting of beta-lactamase, OmpA protein, and that of carboxyeptidase G2 from Pseudomonas. Translation may be coupled to translation initiation for any of the E. coli proteins as well as to initiation sites for any of the highly expressed proteins of the host to cause intracellular expression of the antigenic polypeptides.

In those cases where restriction minus strains of a host Pseudomonas species are not available, transformation efficiency with plasmid constructs isolated from E. coli are poor. Therefore, passage of the Pseudomonas cloning vector through an r- m+ strain of another species prior to transformation of the desired host, as set forth in Bagdasarian, M., et al., Plasmids of Medical, Environmental and Commercial Importance, pp. 411-422, Timmis and Puhler eds., Elsevier/North Holland Biomedical Press (1979), specifically incorporated herein by reference, is desired.

Furthermore, a preferred expression system in hosts of the genera Bacillus involves using plasmid pUB110 as the cloning vehicle. As in other host vector systems, it is possible in Bacillus to express the antigenic polypeptides of the present invention as either an intracellular or a secreted protein. The present embodiments include both systems. Shuttle vectors that replicate in both Bacillus and E. coli are available for constructing and testing various genes as described by Dubnau, D., Gryczan, T., Contente, S., and Shivakumar, A.G. in Genetic Engineering, Vol. 2, Setlow and Hollander eds., Plenum Press, New York, New York, pp. 115-131, (1980), specifically incorporated herein by reference. For the expression and secretion of antigenic polypeptides from B. subtilis, the signal sequence of alpha-amylase is preferably coupled to the coding region for the antigenic polypeptide. For synthesis of intracellular polypeptides, the portable DNA sequence will be translationally coupled to the ribosome binding site of the alpha-amylase leader sequence.

Transcription of either of these constructs is preferably directed by the alpha-amylase promoter or a derivative thereof. This derivative contains the RNA polymerase recognition sequence of the native alpha-amylase promoter but incorporates the lac operator region as well. Similar hybrid promoters constructed from the penicillinase gene promoter and the lac operator have been shown to function in Bacillus hosts in a regulatable fashion as set forth by Yansura, D.G. and Henner in Genetics and Biotechnology of Bacilli, Ganesan, A.T. and Hoch, J.A., eds., Academic Press, pp. 249-263, (1984), specifically incorporated by reference. The lacI gene of lacI^{q} also would be included to effect regulation.

One preferred construction for expression in Clostridium is in plasmid pJU12 described by Squires, C. H. et al in J. Bacteriol. 159:465-471 (1984), specifically incorporated herein by reference, transformed into C. perfringens by the method of Heefner, D. L. et al. as described in J. Bacteriol. 159:460-464 (1984), specifically incorporated herein by reference. Transcription is directed by the promoter of the tetracycline resistance gene. Translation is coupled to the Shine-Dalgarno sequences of this same tet^{r} gene in a manner strictly analogous to the procedures outlined above for vectors suitable for use in other hosts.

Maintenance of foreign DNA introduced into yeast can be effected in several ways. See, for example, Botstein, D., and Davis, R. W., in The Molecular Biology of the Yeast Saccharomyces, Cold Spring Harbor Laboratory, Strathern, Jones and Broach, eds., pp. 607-636 (1982), specifically incorporated herein by reference. One preferred expression system for use with host organisms of the genus Saccharomyces harbors the antigenic polypeptide gene on the 2 micron plasmid. The advantages of the 2 micron circle include relatively high copy number and stability when introduced into cir° strains. These vectors perferably incorporate the replication origin and at least one antibiotic resistance marker from pBR322 to allow replication and selection in E. coli. In addition, the plasmid will preferably have 2 micron sequences and the yeast LEU2 gene to serve the same purposes in LEU2 mutants of yeast.

The regulatable promoter from the yeast GALl gene will preferably be adapted to direct transcription of the antigenic polypeptide gene. Translation of the DNA sequence in yeast will be coupled to the leader sequence that directs the secretion of yeast alpha-factor. This will cause formation of a fusion protein which will be processed in yeast and result in secretion of the desired antigenic polypeptide. Alternatively, a methionyl-antigenic polypeptide will be translated for inclusion within the cell.

As will be seen from an examination of the individual cloning vectors and systems contained in Table I and description, various operational elements may be present in each of the preferred vectors of the present invention. It is contemplated any additional operational elements which may be required may be added to these vectors using methods known to those of ordinary skill in the art, particularly in light of the teachings herein.

In practice, it is possible to construct each of these vectors in a way that allows them to be easily isolated, assembled, and interchanged. This facilitates assembly of numerous functional genes from combinations of these elements and the coding region of the antigenic polypeptide. Further, many of these elements will be applicable in more than one host.

At least one origin of replication recognized by the contemplated host microorganism, along with at least one selectable marker and at least one promoter sequence capable of initiating transcription of the DNA encoding for the antigenic polypeptide are contemplated as being included in these vectors. It is additionally contemplated that the vectors, in certain preferred embodiments, will contain DNA sequences capable of functioning as regulators ("operators"), and other DNA sequences capable of coding for regulator proteins. In preferred vectors of this series, the vectors additionally contain ribosome binding sites, transcription terminators and leader sequences.

These regulators, in one embodiment, will serve to prevent expression of the DNA sequence encoding for the antigenic polypeptide in the presence of certain environmental conditions and, in the presence of other environmental conditions, allow transcription and subsequent expression of the protein coded for by the DNA sequence. In particular, it is preferred that regulatory segments be inserted into the vector such that expression of the DNA sequence will not occur in the absence of, for example, isopropylthiobeta -d-galactoside. In this situation, the transformed microorganisms containing the DNA of interest may be grown to a desired density prior to initiation of the expression of the antigenic polypeptides. In this embodiment, expression of the desired antigenic polypeptide is induced by addition of a substance to the microbial environment capable of causing expression of the DNA sequence after the desired density has been achieved.

Additional operational elements include, but are not limited to, ribosome-binding sites and other DNA sequences necessary for microbial expression of foreign proteins. The operational elements as discussed herein can be routinely selected by those of ordinary skill in the art in light of prior literature and the teachings contained herein. General examples of these operational elements are set forth in B. Lewin, Genes, Wiley & Sons, New York (1983), which is specifically incorporated herein by reference. Various examples of suitable operational elements may be found on the vectors discussed above and may be elucidated through review of the publications discussing the basic characteristics of the aforementioned vectors.

In one preferred embodiment of the present invention, an additional DNA sequence is located immediately preceding the DNA sequence which codes for the antigenic polypeptide. The additional DNA sequence is capable of functioning as a translational coupler, i.e., it is a DNA sequence that encodes an RNA which serves to position ribosomes immediately adjacent to the ribosome binding site of the antigenic polypeptide RNA with which it is contiguous.

Upon synthesis and/or isolation of all necessary and desired component parts of the above-discussed cloning vectors, the vectors are assembled by methods generally known to those of ordinary skill in the art. Assembly of such vectors is believed to be within the duties and tasks performed by those with ordinary skill in the art and, as such, is capable of being performed without undue experimentation. For example, similar DNA sequences have been ligated into appropriate cloning vectors, as set forth in Schonert et al., Proceedings of the National Academy of Sciences U.S.A., 81:5403-5407 (1984), which is specifically incorporated herein by reference.

In construction of the cloning vectors of the present invention, it should additionally be noted that multiple copies of the DNA sequence encoding for the antigenic polypeptide and its attendant operational elements may be inserted into each vector. In such an embodiment, the host organism would produce greater amounts per vector of the desired antigenic polypeptides. The number of multiple copies of the DNA sequence which may be inserted into the vector is limited only by the ability of the resultant vector, due to its size, to be transferred into and replicated and transcribed in an appropriate host microorganism.

Additionally, it is preferred that the cloning vector contain a selectable marker, such as a drug resistance marker or other marker which causes expression of a selectable trait by the host microorganism. Such a drug resistance or other selectable marker is intended in part to facilitate in the selection of transformants. Additionally, the presence of such a selectable marker on the cloning vector may be of use in keeping contaminating microorganisms from multiplying in the culture medium. In this embodiment, such a pure culture of the transformed host microorganisms would be obtained by culturing the microorganisms under conditions which require the induced phenotype for survival.

It is noted that, in preferred embodiment, it is also desirable to reconstruct the 3' end of the coding region to allow assembly with 3' non-translated sequences. Included among these non-translated sequences are those which stabilize the mRNA or enhance its transcription and those that provide strong transcriptional termination signals which may stabilize the vector as they are identified by Gentz, R., Langner, A., Chang, A.C.Y., Cohen, S.H., and Bujard, H. in Proc. Natl. Acad. Sci. USA 78:4936-4940 (1981), specifically incorporated herein by reference.

The vector thus obtained is then transferred into the appropriate host microorganism. It is believed that any microorganism having the ability to take up exogenous DNA and express those genes and attendant operational elements may be chosen. It is preferred that the host microorganism be an anaerobe, facultative anaerobe or aerobe. Particular hosts which may be preferable for use in this method include yeasts and bacteria. Specific yeasts include those of the genus Saccharomyces, and especially Saccharomyces cerevisiae.

Specific bacteria include those of the genera Bacillus and Escherichia and Pseudomonas. Various other preferred hosts are set forth in Table I, supra. In other, alternatively preferred embodiments of the present invention, Bacillus subtilis, Escherichia coli or Pseudomonas aeruginosa are selected as the host microorganisms.

After a host organism has been chosen, the vector is transferred into the host organism using methods generally known by those of ordinary skill in the art. Examples of such methods may be found in Advanced Bacterial Genetics by R. W. Davis et al., Cold Spring Harbor Press, Cold Spring Harbor, New York, (1980), which is specifically incorporated herein by reference. It is preferred, in one embodiment, that the transformation occur at low temperatures, as temperature regulation is contemplated as a means of regulating gene expression through the use of operational elements as set forth above. In another embodiment, if osmolar regulators have been inserted into the vector, regulation of the salt concentrations during the transformation would be required to insure appropriate control of the synthetic genes.

If it is contemplated that the recombinant antigenic polypeptides will ultimately be expressed in yeast, it is preferred that the cloning vector first be transferred into Escherichia coli, where the vector would be allowed to replicate and from which the vector would be obtained and purified after amplification. The vector would then be transferred into the yeast for ultimate expression of the antigenic polypeptide.

The host microorganisms are cultured under conditions appropriate for the expression of the antigenic polypeptide. These conditions are generally specific for the host organism, and are readily determined by one of ordinary skill in the art, in light of the published literature regarding the growth conditions for such organisms, for example Bergey's Manual of Determinative Bacteriology, 8th Ed., Williams & Wilkins Company, Baltimore, Maryland, which is specifically incorporated herein by reference.

Any conditions necessary for the regulation of the expression of the DNA sequence, dependent upon any operational elements inserted into or present in the vector, would be in effect at the transformation and culturing stages. In one embodiment, the cells are grown to a high density in the presence of appropriate regulatory conditions which inhibit the expression of the DNA sequence encoding for the antigenic polypeptide. When optimal cell density is approached, the environmental conditions are altered to those appropriate for expression of the DNA sequence. It is thus contemplated that the production of the antigenic polypeptide will occur in a time span subsequent to the growth of the host cells to near optimal density, and that the resultant antigenic polypeptide will be harvested at some time after the regulatory conditions necessary for its expression were induced.

The transcription terminators contemplated herein serve to stabilize the vector. In particular, those sequences as described by Gentz et al., in Proc. Natl. Acad. Sci. USA 78: 4936-4940 (1981), specifically incorporated herein by reference, are contemplated for use in the present invention.

It is to be understood that application of the teachings of the present invention to a specific problem or environment will be within the capabilities of one having ordinary skill in the art in light of the teachings contained herein. Examples of the products of the present invention and representative processes for their isolation and manufacture appear in the following examples. It should be noted that all literature references used to further elucidate these examples are specifically incorporated herein by reference.

### EXAMPLES

The literature articles cited herein are incorporated by reference in their entirety.

### RI. CONSTRUCTION OF THE LAMBDA GT11 EXPRESSION LIBRARY

### A. Construction of The M. Hyopneumoniae Genomic DNA Expression Library

The rationale for construction of a genomic expression library was to obtain a representative clone for every antigenically active protein that could be expressed by M. hyopneumoniae. Since prokaryotic DNA does not contain introns, it was not necessary to construct a cDNA library to accomplish this. Because of the small genome size, a relatively small number of clones are required to adequately represent the M. hyopneunoniae genome. Using as the estimate of the genome size for M. hyopneumoniae, it was calculated that for a 99% probability of having every 100 bp region in both orientations and in all three reading frames, 1.4 x 10⁵ individual recombinants were required.

In lambda gt11, cloned DNA fragments coding for peptides are expressed as fusion proteins when they are inserted at the unique EcoRI site near the carboxy terminus of the beta-galactosidase gene in the phage. Insertion of foreign DNA in the beta-galactosidase gene inactivates the gene, thus allowing identification of recombinant phages on indicator plates.

The M. hyopneumoniae genomic expression library was obtained in four consecutive steps. First, genomic DNA was obtained from M. hyopneumoniae cells. Second, random fragments were generated by sonication, the ragged ends were blunted using phage T4 polymerase, internal EcoRI methylase, EcoRI linkers were added to the ends and excess linkers cleaved off. Third, the prepared fragments were ligated into the lambda gt11 vector DNA (purchased from Vector Cloning Systems, San Diego, CA) and packaged in vitro. Finally, the in vitro packaged recombinant phage were amplified.

### 1. Preparation of Genomic DNA from Mycoplasma hyopneumoniae cells

Approximately 10¹¹ frozen M. hyopneumoniae cells were thawed in ice and transferred to a polypropylene tube. The volume of cells was 0.8 ml. To this was added a ml of proteinase K solution: 0.075 M Tris pH8; 0.17 M EDTA pH8; 0.15% Triton® X 100; and 400 ug/ml Proteinase K (Boehringer Mannheim Biochemicals, Indianapolis, Indiana). The cells were incubated with the proteinase K solution at 65°C for 30 minutes. 2.0 ml of phenol was added to the mixture and the tube was gently rocked at 4°C for 20 min. The sample was then centrifuged in a Beckman JA20 rotor at 5000 rpm for 5 min at 4°C. The aqueous phase was removed with a large bore plastic pipette. The aqueous phase was extracted with 2 ml of a 24:1 mixture of chloroform and isoamyl alcohol, by rocking for 20 min at 4°C, letting the tube stand 5 min for the phases to separate, and then removing the aqueous phase with a large bore plastic pipette.

The aqueous phase was then re-extracted with phenol followed by chloroform 1 isoamyl alcohol as described in the above paragraph. The extracted aqueous phase was adjusted to 0.3 M sodium acetate by adding a 1/10 volume of a 3.0 M sodium acetate stock solution. DNA was precipitated from this solution by adding 2.5 volumes of cold ethanol. Gentle mixing with the ethanol yielded a large, viscous mass of nucleic acid that was "spooled" out of solution using a small glass rod. This material was then rinsed twice with 70% ethanol and allowed to air-dry. The dry nucleic acids were resuspended in 0.5 ml of TE buffer by rocking the solution for 16 hr at 4°C.

In order to remove any contaminating RNA from the M. hyopneumoniae DNA preparation, the nucleic acids were treated with RNaseA. Five-tenths milliliter of the sporozoite nucleic acid preparation was treated with 2.5 ug/ml of RNaseA (Miles Laboratories, Elkhart, Indiana) at 65°C for 30 min. The aqueous phase was adjusted to 3.2 M ammonium acetate by adding a 0.75 volume of a 7.5 M stock solution. The DNA was precipated from this solution with the addition of 0.54 volumes of isopropanol. Mixing of the isopropanol yielded a large viscous pellet that was spooled from the solution and rinsed twice with 80% ethanol. After air drying, the DNA was resuspended in 0.2 ml of TE buffer.

Integrity of the M. hyopneumoniae DNA was analyzed by electrophoresis through an agarose gel and subsequent staining of the gel with ethidium bromide. These results indicated that the DNA was of high molecular weight and relatively free of RNA. The concentration of the DNA was determined by optical absorbance at 260 nm. This particular preparation yielded 90 ug of purified DNA.

### 2. Preparation of Sonicated M. hyopneumoniae Genomic DNA Fragments

In order to have a given fragment from the M. hyopneumoniae genome aligned in the proper reading frame for transcription and translation of the native polypeptide, it was decided to generate random fragments by sonication to that all possible frames would be represented.

M. hyopneumoniae DNA was sonicated with a small tipped probe of a Branson Sonifier cell disrupter 200 set at the lowest power setting. 80 Ug of DNA in 200 Ul total volume was sonicated in three 3-second bursts. This generated fragments ranging in size from 0.3 to 23 Kb in size with the highest proportion in the 1.3 to 4.4 Kb range.

The ragged ends generated by the sonication were blunted using T4-DNA polymerase (New England Biolabs; Beverly, Mass.). The reaction was carried out in 33 mM Tris Acetate pH7.8; 66 mM Potassium Acetate, 10 mM Magnesium Acetate, 0.1 mg/ml Bovine Serum Albumin, 0.5 mM dithiothreitol, and 0.1 mM of each of the deoxynucleotide triphosphates, dATP, dGTP, dCTP, dTTP. For 65 Ug of sonicated DNA, 20 units of T4 polymerase was reacted for 1 hour at 37°. The reaction was stopped by heating the sample for 10 min at 68°C. Excess salts were removed by passing the reaction mix over a Biogel P30 (Bio Rad) column equilibrated in TE.

In order to protect any M. hyopneumoniae genomic DNA fragments that might have internal EcoRI sites, it was necessary to modify the DNA. EcoRI methylase (New England Biolabs) was reacted with the fragmented M. hyopneumoniae DNA in the presence of S-adenosyl methionine using conditions recommended by the supplier. The methylase was inactivated by phenol extraction of the reaction mix, and residual phenol was removed by ether extractions. The mix was then adjusted to 0.3 M with sodium acetate, and 2.5 volumes of ethanol were added. After 30 min at -70°C, the precipitated DNA was pelleted by centrifugation. This DNA was resuspended in TE buffer.

Short oligonucleotide EcoRI linkers (New England Biolabs) were added to the blunt ended fragments in a ligation reaction consisting of 66 mM Tris pH7.6, 5 mM MgCl₂, 5mM dithiothreitol, 10 mM ATP, with the linker at 5-10 uM. T4 DNA ligase (P.L. Biochemicals) was added and the reaction proceeded at 14°C for 16 hours. The ligation reaction was terminated by heating the mixture at 70° for 10 min.

Sodium chloride was then added to 0.1 M, excess EcoRI was added, and the reactions were incubated at 37°C for several hours. The EcoRI was added, and the reactions were incubated at 37°C for several hours. The EcoRI was inactivated by heating at 70°C for 10 min.

### 3. Ligation of Fragments to lambda gt11 and In Vitro Packaging of the Recombinant DNA

Phosphatased and EcoRI-cleaved lambda gt11 DNA was mixed with the prepared fragments of M. hyopneumoniae DNA. These DNAs were ligated with T4 DNA ligase (P.L Biochemicals) overnight at 14°C. A small aliquot of the ligation reaction mixture was analyzed by gel electrophoresis to monitor the ligation reaction. The mixture was heated at 70°C for 5 min and then mixed with lambda in vitro packaging extracts -(Vector Cloning Systems, San Diego, CA). The packaging reaction was allowed to proceed for 60min at room temperature and then a drop of chloroform was added to prevent bacterial growth. Titration of this mix yielded a library with a complexity of 1.5 X 10⁵ recombinants.

### 4. Amplification of the M. hyopneumoniae Expression Library

Packaged phage were diluted with lambda oil and adsorbed to E. coli strain Y1088 as described by Young, R. and Davis, R. in Science 222:778-782 (1983). Amplification of the library on this strain ensures that the beta-galactosidase gene is not expressed; therefore, any phage containing coding sequences that might be deleterious to the host E. coli cell are not expressed and not lost from the library. Amplifications of the library yield a stock that was 6 X 10⁹ phage per ml.

Removal of excess linkers and size fractionation of the DNA fragments was carried out by density centrifugation in a 10 to 40% sucrose gradient in 1M NaCl; 20 mM tris pH8, 5 mM EDTA. The DNA was applied to the top of the gradient and was spun in a Beckman SW41 rotor at 26,000 rpm for 24 hours at 15°C.

Fractions were collected in 0.3 ml aliquots. These were assayed by aqueous gel electrophoresis and staining by Ethidium Bromideto compare the size of the smear of fragments in each fraction with molecular weight markers. Fractions which were enriched for fragments in the 1 to 6 Kb range were pooled, then dialyzed and concentrated using a Centricon 30 (Amicon) apparatus.

### II. GENERAL METHODOLOGY

### A. Antibody Screening of the Lambda GT11: M. hyopneumoniae Expression Library

The lambda gt11:M. hyopneumoniae expression library was plated at densities of 5,000 through 20,000 phage per 150-mm plate using E. coli Y1090 as host as described by Young and Davis in Science 222:778-782, (1983). Plates were incubated at 37° or 42° for 4 hr, then overlaid with nitrocellulose filters (BA-85, Schleicher and Schuell) that had been soaked in 10 mM IPTG and air-dried. After incubating overnight at 37°, filters were batch-washed 3 x 10 min in TBS (10 mM Tris-HCl pH 8.0, 150 mM NaCl). Non-specific protein binding sites on the filters were blocked by incubating filters for 60 min in TBS + 2% bovine serum albumin (Fraction V, Miles Laboratories, Elkhart, IN). The filters were then incubated individually or in pairs for 2 hr with 10-20 ml of primary antibody (e.g., immune swine serum, hyperimmune rabbit anti-mycoplasma serum, mouse monoclonal anti-mycoplasma antibodies) typically diluted 1:200 (1:500 for monoclonals) with TBS to which had been added 2% bovine serum albumin (BSA). The filters were washed 3 x 10 min with TBS containing 0.1% NP-40 (Sigma), then incubated singly or in pairs for 60 min with 10-20 ml solution of a second antibody (e.g., peroxidase-conjugated goat anti-rabbit IgG, Cappel Laboratories) diluted 1:500 in TBS + 2% bovine serum albumin. Filters were batch-washed 3 x 10 min in TBS and stained in a solution comprising 200 ml TBS, 2.5 ml hydrogen peroxide and 40 ml of a 3 mg/ml solution of 4-chloro-1-napthol in methanol. Staining was quenched by removing the filters to water. Positive staining plaques were subjected to several rounds of rescreening with antibody as described above until pure.

### B. Elution Of Antibodies Bound To Proteins Immobilized On Nitrocellulose Membranes

When proteins are immobilized on nitrocellulose membranes such as Western blot transfers of proteins separated on polyacrylimide gels or replicas of phage plaques taken from agar plates, it is possible to bind antibodies which specifically recognize the immobilized proteins. Antibodies which do not specifically bind to the immobilized proteins remain in solution and can be easily washed away, leaving behind only those specifically bound.

The bound antibodies can be eluted by rinsing the filters in a low pH buffer (5 mM glycine, pH 2.3, 0.5 M NaCl, 0.5% Tween® 20, 0.01% BSA), which dissociates the antibody-antigen complex. If the eluted antibodies are immediately neutralized,i.e., using a 50 mM Tris HCl, final concentration, they retain full activity and can be used for a variety of analytical purposes.

### 1. Determination of Mycoplasma Protein Corresponding to Insert Clone

Antibodies eluted from plaque replicas of a purified recombinant clone were used to determine which mycoplasma protein corresponded to that clone. By eluting antibodies bound to plaque replicas of the recombinant clones and using those antibodies to probe Western blots of mycoplasma proteins, it was possible to determine which protein is encoded in the recombinant clone.

Five thousand to ten thousand plaques from a single purified recombinant clone were plated on a 100 mm plate, and a plaque lift was made. The lift filters were washed 3 x 10 min in TBS and non-specific protein binding was blocked by incubation in TBS + 10% BSA for 1 hr. The filters were washed 3 x 10 min in TBS. A strip 5 mm wide was cut from the center of the filter disc. Polyclonal anti-mycoplasma serum was bound to the strip, washed and eluted by Western blot elutions. The eluted antibodies were then used to probe a Western blot of mycoplasma proteins.

### III. PLASMID VECTORS FOR EXPRESSION OF FUSION PROTEINS

A number of antigenically reactive M. hyopneumoniae recombinant phage clones were identified in the expression library. Since the lambda gt11 lysogens appeared to make a limited quantity of fusion protein, we constructed a plasmid expression vector that would produce the fusion proteins in milligram quantities.

### A. pSEV4

Plasmid pLG2 was obtained from Dr. L. Guarente (MIT) (Guarente, L., in Cell, 20: 543-553 (1980). This vector is a pBR322 derivative which, like lambda gt11, has lac operator and promoter sequenes in addition to a wild-type beta-galactosidase gene containing a single Eco RI site near the 3' end of the gene. In addition, pLG2 contains the lac repressor gene. Moving M. hyopneumoniae DNA inserts from lambda gt11 into the EcoRI site of this vector yields an identical fusion protein to that initially identified in the phage.

Plasmid pLG2 was modified to remove an extra EcoRI site prior to its use for expression. Plasmid pLG2 was partially digested with EcoRI restriction endonuclease to linearize the plasmid. The plasmid DNA was then displayed on a preparative agarose gel and the linear-sized DNA band was eluted from the gel. The eluted DNA was precipitated by making the solution 0.3 M with sodium acetate and adding 2.5 volumes of ethanol. The DNA was pelleted by centrifugation and the pellet was resuspended in TE buffer. The Klenow fragment of E. coli DNA Polymerase I was mixed with the DNA in the presence of dATP and dTTP to fill in the EcoRI cohesive ends. After heat inactivation at 70°C for 10 min, T4 DNA ligase (P.L. Biochemicals) was added and the mixture was incubated at 4°C for 16 hr. The ligated DNA was then used to transform E. coli AMA1004. Casadaban, M., et al., in Methods in Enzymology 100:293 (1983).

Transformants were selected on ampicillin plates in the presence of a chromagenic substrate for beta-galactosidase activity (X-GAL). Transformants with beta-galactosidase activity were screened by cleaving the DNA with EcoRI. A plasmid, pSEV4, that had only a single EcoRI site near the amino terminus of the beta-galactosidase gene was identified from the transformants and characterized.

Plasmid pSEV4 has a unique EcoRI site near the amino terminus of the beta-galactosidase gene. Plasmid pSEV4 contains the wild-type lac operator, promoter and repressor in addition to the beta-galactosidase gene. Upon induction with IPTG for 60 min, beta-galactosidase activity was increased by 300-fold. Uninduced cells containing pSEV4 produced approximately 1000 units/mg of total cellular protein, whereas IPTG-induced cells gave approximately 300,000 units/mg of total cell protein. Protein gel analysis of induced and uninduced cells also showed the overproduction of beta-galactosidase by induced cells. This new plasmid, pSEV4, has been used to express a number of M. hyopneumoniae antigens as fusion proteins.

### B. pSEV6

A successor to pSEV4 was constructed to allow polarized "cassette" subcloning of DNA inserts from lambda gtll directly into a plasmid expression vector. Because EcoRI inserts could be subcloned in either orientation in pSEV4, each pSEV4 subclone for its antigenic reaction. Polarized subcloning using pSEV6 obviates the need for this extra analysis.

Extensive mapping of pSEV4 located five restriction endonuclease sites in the lac operon 5' to the beta-galactosidase gene's unique EcoRI site. Three of these sites are unique and two were made unique by deletion of the superfluous DNA between the lacI gene and the pBR322-derived amp^{r} gene. Only one useful restriction site was found 3' to the EcoRI site, the Ncol site, therefore, additional restriction enzyme sites were inserted in this region using a chemically synthesized polylinker.

The construction of pSEV6 was done in two steps. First, pSEV4 was shortened by approximately 5,700 bp to eliminate superfluous DNA. Plasmid pSEV4 was cleaved with SphI restriction endonuclease, and the enzyme was inactivated by heating at 70°C for 10 min. The DNA was then partially digested with AatII and the resulting digest was displayed by electrophoresis on a preparative agarose gel. The 7,620 bp fragment was excised from the gel and electroluted.

The electroluted DNA was precipitated from a 0.3 M sodium acetate solution by adding 2.5 volumes of ethanol and incubating at -70°C for 30 min. The DNA was concentrated by pelleting in a Brinkman micro -- centrifuge for 15 min and the pellet was resuspended in TE buffer. T4 DNA polymerase (New England Biolabs) was added to blunt-end the cohesive ends generated by the AatII digest. After heat inactivation of the T4 DNA polymerase, T4 DNA ligase (P.L. Biochemicals) was added to ligate the blunt ends of the DNA fragment. The ligated DNA was used to transform competent AMA1004 E. coli cells. Lac⁺ transformants were screened for the 7,620 bp plasmid. One plasmid, pSEV5, was identified and characterized as having the appropriate structure.

DNA from pSEV5 was purified by standard methods, and subsequently cleaved with the restriction endonuclease NcoI, which cleaved at a unique site 3' of the beta-galactosidase gene. An oligonucleotide adapter molecule would regenerate the NcoI site and which also contained BglII and Kpn I sites was chemically synthesized. The sequence of this adapter molecule is as follows:

5'-GTAAGGAGGAATAACATATGGAATTCGAG-3'
3' -ACGTCATTCCTCCTTATTGTATACCTTAAGCTCCTAG -5'

This oligonucleotide was ligated to the NcoI cleaved pSEV5 with T4 DNA ligase (P.L. Biochemicals). The ligated DNA was used to transform competent E. coli AMA1004 cells. DNA from the resulting lac⁺ transformants was screened for the presence of the unique KpnI and NcoI sites. A plasmid was identified from this screen with all of the designed sequences. This plasmid, pSEV6, has been used for expression of various of the antigenically reactive fusion proteins.

### IV. PURIFICATION OF THE CLONED ANTIGENS FOR ANIMAL TESTING

The beta-galactosidase::M. hyopneumoniae antigen fusion proteins have been purified either by use of a substrate analog affinity column for beta-galactosidase or by classical methods of protein purification.

### A. Preparation of Extracts

Two liters of Luria broth, pH 7.5, containing 50 ug/mL of ampicillin were inoculated with 10-20 ml of an overnight culture of E. coli AMA1004 containing one of the recombinant plasmids. The cells were allowed to grow at 37°C to mid-log phase (A₆₀₀ = 0.2). Isopropyl-thiogalactoside (IPTG) was added to a final concentration of 1 mM to induce formation of the fusion protein. The cells were allowed to grow out for 2 hours, and then harvested by centrifugation at 5000 x G for 15 min at 4°C. All subsequent operations were carried out at 4°C.

The cells were resuspended in 20 mL of breaking buffer (50 mM Tris-HCl, pH 7.5, 250 mM NaCl, 10 mM MgCl₂, 5% glycerol, 1 mM phenymethyl sulfonyl fluoride (PMSF)) at 4°C and centrifuged again at 5000 x G for 15 min. The cells were again suspended in 20-40 mL of breaking buffer. The cells could be frozen at this point and stored at -20°C if desired.

The unfrozen or thawed cells were broken with two passes through a French pressure cell (Aminco) at 20,000 psi. Cell debris was removed by centrifugation at 30,000 x G for 30 min. Further clarification of the extract could be obtained at this point by ultracentrifugation at 100,000 x G for 30 min. The fusion protein was then precipitated by the addition of ammonium sulfate to a final concentration of 20 to 40% saturation. The optimal concentration of ammonium sulfate required for precipitation of the fusion protein varies with the individual protein and must be determined experimentally, using for example, procedures set forth in Heppel, L. in Methods in Enzymology, 1:570-576 (1955).

The precipitate solution was stirred for one hour and the precipitate was removed by centrifugation at 30,000 x G for 15 min. The pellet was redissolved in 10 to 15 ml of starting buffer (50 mM Tris-HCl, pH 7.5, 250 mM NaCl, 10 mM MgCl₂, 1 mM dithiothreitol (DTT) and 0.1% Triton® X-100), and then dialyzed overnight against 500 mL of starting buffer.

### 1. Affinity Purification Procedure

The use of a beta-galactosidase affinity column is based on the method described by Steers and Cuatrecasas in Methodsin Enzymology, 34:350-358 (1974). Affinity resin (p-aminophenyl-beta-D-Thiogalactopyranoside-agarose, obtained from Sigma) was packed into a 1.5 cm diameter by 15 cm column. The column was washed with 10 column volumes of starting buffer of 50 mM Tris-HCl, pH 7.5, 250 mM NaCl, 10 mM MgCl₂, and 1.0 mM dithiothreitol (DTT) and 0.1 Triton®-X100 before use. The column can be regenerated after use by washing extensively with elution buffer 0.1 sodium borate, pH 10.0, 250 mM NaCl, 1 mM DTT or by washing with 6M guanidine hydrochloride in 50 mM Tris-HCl, pH 7.5. After washing, the column is reequilibrated with 10 column volumes of starting buffer.

For affinity chromatography, dialyzed material was applied to the pre-equilibrated affinity column at a flow rate of about 0.2 ml/min. After the sample was applied to the column, the column was washed with 15 ml of starting buffer at the same flow rate, then with 30 ml of starting buffer at about 0.5 ml/min followed with 180 ml starting buffer at about 1 ml/min. Finally, the column was washed with 120 ml of starting buffer without Triton at the same flow rate.

The absorbed protein was eluted with 0.1 sodium borate, pH 10.0, 250 mM NaCl, 1 mM DTT using 120 ml at a flow rate of about 1 ml/min. The peak-protein containing fractions are pooled and could be concentrated if desired to about 10 ml using an Amicon ultrafiltration device (Model 8050) containing an YM-30 membrane.

### 2. Ultracentrifuge Purification

An alternative purification usable for some of the fusion proteins (e.g., pSEV4::CH2-13) is accomplished by obtaining dialyzed protein as set forth above. The dialyzed material is subjected to ultracentrifugation at 100,000 x G for 30 min. The pellet containing the bulk of the fusion protein was redissolved in a small volume of dialysis buffer 50 mM Tris-HCl, ph 7.5, 250 mM NaCl, 10 mM MgCl2 and 1.0 mM dithiothreitol (DTT) and 0.1 M Triton®-X 100. This method yields material that is not as pure as that generated by the affinity column when judged by SDS-polyacrylamide electrophoresis.

### 3. Analysis

The purified materials obtained by these methods were analyzed for protein by the Bio-Rad (Richmond, CA) protein method as recommended by the manufacturer. It was also subjected to analysis by SDS-polyacrylamide electrophoresis. These gels are visualized either by protein staining or by Western blot analysis. Protein staining was typically done with either the silver stain method as described by Wray, W.P. et al. in Anal. Blockem. 118:197-203 (1981) or the Bio-Rad protein stain. Western blot analysis is carried out as described by Remart, J. et al. in PNAS (USA) 76:3116 (1979).

The Western blot analysis involves electrophoretic transfer of the resolved protein bands to nitrocellulose, blocking the nitrocellulose paper with BSA, probing with a specific antibody (either anti-betagalactosidase or anti-mycoplasma sera). After washing, the blots are probed with the appropriate peroxidase conjugated second antibody, followed by color development using the peroxidase catalyzed reaction.

### V. PROCEDURES FOR OBTAINING THE ENTIRE M. hyopneumuniae GENE ENCODED BY A FUSION PROTEIN CLONE

The recombinant M. hyopneumoniae clones that were picked as reactive with various anti-mycoplasma sera contain only a portion of the entire coding regin for that particular polypeptide, because of the requirementthat the insert sequence be in frame with the beta-galactosidace gene of lambda gtll and the limited number of clones screened. In some cases it may be important to have cloned the entire coding sequence of a given antigenic M. hyopneumoniae polypeptide in order to maximize the immune response or modulate the response. A method isoutlined below that will allow theisolation of clones containing such full length fragments.

The lambda gt11 expression library described above, may also be viewed as a simple genomic library if one does not require that the inserted segments of M. hyopneumoniae be expressed as fusion proteins. Some of these clones should contain the entire coding region for a particular protein even though it is not in frame with the beta galactosidase expression system.

These clones may be detected using DNA hybridization probes derived from the clones already picked by antibody methods and known to correspond to particular mycoplasma proteins. The insert fragments from these clones is 'nick translated' using E- coli DNA polymerase to incorporate 32P-labeled deoxynucleotides into the DNA. This labeled DNA is then used as a radioactive probe to select an homologous M. hyopneumoniae sequence from the recombinant lambda gt11 library. Phages selected from the recombinant library by this method are plaque purified, DNA is prepared, and the M. hyopneumoniae specific insert is mapped with various restriction endonucleases. The resulting map is compared with a similar map derived from the initial clone to confirm the identity of the new genomic phage.

Because there are no introns in the prokaryotic genes, one can determine, from the size of the protein encoded, how much DNA to either side of the labeled DNA must be included to be sure that the entire gene is included. The entire gene may not be contained in a single clone; however, it is possible for anyone skilled in the art to obtain the entire gene. This is done by "walking" along the M. hyopneumoniae genome by isolating phages that contain flanking M. hyopneumoniae genomic DNA using the method by Bender, E. et al. in J. Mol. Biol. 168:17-33 (1983).

### VI. PROCEDURES FOR IDENTIFICATION OF CLONES CORRESPONDING TO SURFACE PROTEINS

Proteins which are exposed on the surface of a mycoplasma cell have been shown to be susceptible to digestion by a protease such as trypsin when whole, intact cells are lightly treated with that enzyme (Klinkect, M., Herrmann, R., and Schaller, H., (1985) Infection and Immunity 49, 329-335) specifically incorporated herein by reference. This technique, in combination with the elution of antibodies from clones, allows the rapid determination of whether a particular clone corresponds to a trypsin sensitive surface protein. Total proteins from trypsinzed and non-trypsinized mycoplasma cells are placed in adjacent lanes and separated by SDS poly-acrylimide slab gel electrophoresis. The displayed proteins are then electroblotted onto nitrocellulose membrane by the western blot procedure. This blot is then probed using antibodies from a polyclonal serum which have been affinity purified from a specificclone using the antibody elution technique described above.

The specific mycoplasma protein on the western blot corresponding to the clone will be revealed by staining of the bound antibody in thelane with proteins from non-trypsinized cells, showing up as a spcific stained band. If that protein is trypsin sensitive, thecorresponding position in the lane with proteins from trypsinized cells will be blank, whereas a non-trypsin sensitive band will stain as in the untreated cells.

## Claims (Claims for the following Contracting State(s): BE, CH, DE, FR, GB, IT, LI, LU, NL, SE)

1. Surface protein of Mycoplasma hyopneumoniae selected from the group consisting of Protein A, Protein C, Protein D and Protein E,
wherein said proteins are recognized by at least one of the following sources of antibody: immune swine serum, hyperimmune rabbit anti-mycoplasma serum, and mouse monoclonal anti-mycoplasma antibodies, and wherein,
said protein A having a molecular weight of 105 kD,
said protein C having a molecular weight of 85 kD,
said protein D having a molecular weight of 70kD, with the proviso that protein p 68 according to EP-A-0196215 is excluded,
and
said protein E having a molecular weight of 43 kD,
the molecular weights of the proteins being determined according to SDS-PAGE.

2. A phage lambda gt11 clone containing a 1.5 kb insert in the EcoRI site of lambda gt11, which insert codes for a portion of the protein A of claim 1.

3. A phage lambda gt11 clone containing a 0.5 kb insert in the EcoRI site of lambda gt11, which insert codes for a portion of the protein C of claim 1.

4. A phage lambda gt11 clone containing a 3.2 kb insert in the EcoRI site of lambda gt11, which insert codes for a portion of the protein D of claim 1.

5. A phage lambda gt11 clone containing a 0.5 kb insert in the EcoRI site of lambda gt11, which insert codes for a portion of the protein E of claim 1.

6. A recombinant DNA method for the manufacture of the proteins of claim 1, which are capable of inducing an antigenic response comprising:
(a) preparation of DNA sequence capable of directing a host micro-organism to produce the protein;
(b) cloning the DNA sequence into a vector capable of being transferred into and replicating in a host micro-organism, such vector containing operational elements for the DNA sequence;
(c) transferring the vector containing the DNA sequence and operational elements into a host micro-organism capable of expressing the antigenic protein;
(d) culturing the host micro-organism under conditions appropriate for amplification of the vector and expression of the protein; and
(e) in either order:
i) harvesting the protein; and
ii) causing the protein to assume a structure whereby it possesses antigenic properties analogous to properties possessed by Mycoplasma organisms.

## Claims (Claims for the following Contracting State(s): AT)

1. Surface protein of Mycoplasma hyopneumoniae selected from the group consisting of Protein A, Protein C, Protein D and Protein E,
wherein said proteins are recognized by at least one of the following sources of antibody: immune swine serum, hyperimmune rabbit anti-mycoplasma serum, and mouse monoclonal anti-mycoplasma antibodies, and wherein, said protein A having a molecular weight of 105 kD,
said protein C having a molecular weight of 85 kD,
said protein D having a molecular weight of 70 kD, with the proviso that protein p 68 according to EP-A-0196215 is excluded,
and said protein E having a molecular weight of 43 kD,
the molecular weights of the proteins being determined according to SDS-PAGE.

2. A phage lambda gt11 clone containing a 1.5 kb insert in the EcoRI site of lambda gt11, which insert codes for a portion of the protein A of claim 1.

3. A phage lambda gt11 clone containing a 0.5 kb insert in the EcoRI site of lambda gt11, which insert codes for a portion of the protein C of claim 1.

4. A phage lambda gt11 clone containing a 3.2 kb insert in the EcoRI site of lambda gt11, which insert codes for a portion of the protein D of claim 1.

5. A phage lambda gt11 clone containing a 0.5 kb insert in the EcoRI site of lambda gt11, which insert codes for a portion of the protein E of claim 1.

6. A recombinant DNA method for the manufacture of the proteins of claim 1, which are capable of inducing an antigenic response comprising:
(a) preparation of DNA sequence capable of directing a host micro-organism to produce the protein;
(b) cloning the DNA sequence into a vector capable of being transferred into and replicating in a host micro-organism, such vector containing operational elements for the DNA sequence;
(c) transferring the vector containing the DNA sequence and operational elements into a host micro-organism capable of expressing the antigenic protein;
(d) culturing the host micro-organism under conditions appropriate for amplification of the vector and expression of the protein; and
(e) in either order:
i) harvesting the protein; and
ii) causing the protein to assume a structure whereby it possesses antigenic properties analogous to properties possessed by Mycoplasma organisms.

7. A method for preparing a phage lambda gt11 clone containing an insert coding for a portion of a surface protein of Mycoplasma hyopneumoniae selected from the group consisting of protein A, protein C, protein D and protein E, wherein a 1.5 kb insert coding for a portion of protein A is inserted into the EcoRI site of lambda gt11, said protein A having a molecular weight of 105 kD the molecular weights being determined by SDS-PAGE.

8. A method for preparing a phage lambda gt11 clone containing an insert coding for a portion of a surface protein of Mycoplasma hyopneumoniae selected from the group consisting of protein A, protein C, protein D and protein E, wherein a 0.5 kb insert coding for a portion of protein C is inserted into the EcoRI site of lambda gt11, said protein C having a molecular weight of 85 kD the molecular weights being determined by SDS-PAGE.

9. A method for preparing a phage lambda gt11 clone containing an insert coding for a portion of a surface protein of Mycoplasma hyopneumoniae selected from the group consisting of protein A, protein C, protein D and protein E, wherein a 3.2 kb insert coding for a portion of protein D is inserted into the EcoRI site of lambda gt11, said protein D having a molecular weight of 70 kD the molecular weights being determined by SDS-PAGE.

10. A method for preparing a phage lambda gt11 clone containing an insert coding for a portion of a surface protein of Mycoplasma hyopneumoniae selected from the group consisting of protein A, protein C, protein D and protein E, wherein a 0.5 kb insert coding for a portion of protein E is inserted into the EcoRI site of lambda gt11, said protein E having a molecular weight of 43 kD the molecular weights being determined by SDS-PAGE.

## Patentansprüche (Patentansprüche für folgende(n) Vertragsstaat(en): BE, CH, LI, DE, FR, GB, IT, LU, NL, SE)

1. Oberflächenprotein von Mycoplasma hyopneumoniae, ausgewählt aus der Gruppe bestehend aus Protein A, Protein C, Protein D und Protein E,
worin diese Proteine durch mindestens eine der folgenden Quellen an Antikörper erkannt werden: Schweineimmunserum, Anti-Mycoplasma-Hyperimmunserum des Kaninchens und monoklonale Anti-Mycoplasma-Antikörper der Maus, und worin
das Protein A ein Molekulargewicht von 105 kD besitzt, das Protein C ein Molekulargewicht von 85 kD besitzt, das Protein D ein Molekulargewicht von 70 kD besitzt, unter der Bedingung, daß das Protein p 68 gemäß EP-A-0196215 ausgenommen ist, und das Protein E ein Molekulargewicht von 43 kD besitzt, wobei die Molekulargewichte der Proteine durch SDS-PAGE bestimmt werden.

2. Ein Phagenklon λgt11, enthaltend ein 1,5 kb-Insert in der EcoRI-Stelle des λgt11, wobei das Insert für einen Teil des Proteins A nach Anspruch 1 kodiert.

3. Ein Phagenklon λgt11, enthaltend ein 0,5 kb-Insert in der EcoRI-Stelle des λgt11, wobei das Insert für einen Teil des Proteins C nach Anspruch 1 kodiert.

4. Ein Phagenklon λgt11, enthaltend ein 3,2 kb-Insert in der EcoRI-Stelle des λgt11, wobei das Insert für einen Teil des Proteins D nach Anspruch 1 kodiert.

5. Ein Phagenklon λgt11, enthaltend ein 0,5 kb-Insert in der EcoRI-Stelle des λgt11, wobei das Insert für einen Teil des Proteins E nach Anspruch 1 kodiert.

6. Ein rekombinantes DNA-Verfahren zum Herstellen der Proteine nach Anspruch 1, die eine Antigenantwort induzieren können, umfassend:
(a) Herstellen einer DNA-Sequenz, die einen Wirtsmikroorganismus veranlassen kann, das Protein herzustellen;
(b) Klonieren der DNA-Sequenz in einen Vektor, der in einen Wirtsmikroorganismus übertragen und darin repliziert werden kann, wobei der Vektor Kontrollelemente für die DNA-Sequenz enthält;
(c) Übertragen des Vektors, enthaltend die DNA-Sequenz und die Kontrollelemente, in einen Wirtsmikroorganismus, der das antigene Protein exprimieren kann;
(d) Kultivieren des Wirtsmikroorganismus unter Bedingungen, die geeignet sind zur Amplifikation des Vektors und zur Expression des Proteins; und
(e) in beliebiger Reihenfolge:
i) Ernten des Proteins; und
ii) das Protein veranlassen, eine Struktur anzunehmen, wodurch es antigene Eigenschaften gewinnt, die analog sind zu den Eigenschaften, die Mycoplasma-Organismen aufweisen.

## Patentansprüche (Patentansprüche für folgende(n) Vertragsstaat(en): AT)

1. Oberflächenprotein von Mycoplasma hyopneumoniae, ausgewählt aus der Gruppe bestehend aus Protein A, Protein C, Protein D und Protein E,
worin diese Proteine durch mindestens eine der folgenden Quellen an Antikörper erkannt werden: Schweineimmunserum, Anti-Mycoplasma-Hyperimmunserum des Kaninchens und monoklonale Anti-Mycoplasma-Antikörper der Maus, und worin
das Protein A ein Molekulargewicht von 105 kD besitzt, das Protein C ein Molekulargewicht von 85 kD besitzt, das Protein D ein Molekulargewicht von 70 kD besitzt, unter der Bedingung, daß das Protein p 68 gemäß EP-A-0196215 ausgenommen ist, und das Protein E ein Molekulargewicht von 43 kD besitzt, wobei die Molekulargewichte der Proteine durch SDS-PAGE bestimmt werden.

2. Ein Phagenklon λgt11, enthaltend ein 1,5 kb-Insert in der EcoRI-Stelle des λgt11, wobei das Insert für einen Teil des Proteins A nach Anspruch 1 kodiert.

3. Ein Phagenklon λgt11, enthaltend ein 0,5 kb-Insert in der EcoRI-Stelle des λgt11, wobei das Insert für einen Teil des Proteins C nach Anspruch 1 kodiert.

4. Ein Phagenklon λgt11, enthaltend ein 3,2 kb-Insert in der EcoRI-Stelle des λgt11, wobei das Insert für einen Teil des Proteins D nach Anspruch 1 kodiert.

5. Ein Phagenklon λgt11, enthaltend ein 0,5 kb-Insert in der EcoRI-Stelle des λgt11, wobei das Insert für einen Teil des Proteins E nach Anspruch 1 kodiert.

6. Ein rekombinantes DNA-Verfahren zum Herstellen der Proteine nach Anspruch 1, die eine Antigenantwort induzieren können, umfassend:
(a) Herstellen einer DNA-Sequenz, die einen Wirtsmikroorganismus veranlassen kann, das Protein herzustellen;
(b) Klonieren der DNA-Sequenz in einen Vektor, der in einen Wirtsmikroorganismus übertragen und darin repliziert werden kann, wobei der Vektor Kontrollelemente für die DNA-Sequenz enthält;
(c) Übertragen des Vektors, enthaltend die DNA-Sequenz und die Kontrollelemente, in einen Wirtsmikroorganismus, der das antigene Protein exprimieren kann;
(d) Kultivieren des Wirtsmikroorganismus unter Bedingungen, die geeignet sind zur Amplifikation des Vektors und zur Expression des Proteins; und
(e) in beliebiger Reihenfolge:
i) Ernten des Proteins; und
ii) das Protein veranlassen, eine Struktur anzunehmen, wodurch es antigene Eigenschaften gewinnt, die analog sind zu den Eigenschaften, die Mycoplasma-Organismen aufweisen.

7. Ein Verfahren zum Herstellen eines Phagenklons λgt11, enthaltend ein Insert, das für einen Teil eines Oberflächenproteins von Mycoplasma hyopneumoniae kodiert, ausgewählt aus der Gruppe bestehend aus Protein A, Protein C, Protein D und Protein E, worin ein 1,5 kb-Insert, das für einen Teil des Proteins A kodiert, in die EcoRI-Stelle von λgt11 eingefügt wird, wobei das Protein A ein Molekulargewicht von 105 kD besitzt, wobei die Molekulargewichte der Proteine durch SDS-PAGE bestimmt werden.

8. Ein Verfahren zum Herstellen eines Phagenklons λgt11, enthaltend ein Insert, das für einen Teil eines Oberflächenproteins von Mycoplasma hyopneumoniae kodiert, ausgewählt aus der Gruppe bestehend aus Protein A, Protein C, Protein D und Protein E, worin ein 0,5 kb-Insert, das für einen Teil des Proteins C kodiert, in die EcoRI-Stelle von λgt11 eingefügt wird, wobei das Protein C ein Molekulargewicht von 85 kD aufweist, wobei die Molekulargewichte der Proteine mit SDS-PAGE bestimmt werden.

9. Ein Verfahren zum Herstellen eines Phagenklons λgt11, enthaltend ein Insert, das für einen Teil eines Oberflächenproteins von Mycoplasma hyopneumoniae kodiert, ausgewählt aus der Gruppe bestehend aus Protein A, Protein C, Protein D und Protein E, worin ein 3,2 kb-Insert, das für einen Teil des Proteins D kodiert, in die EcoRI-Stelle von λgt11 eingefügt wird, wobei das Protein D ein Molekulargewicht von 70 kD besitzt, wobei die Molekulargewichte der Proteine durch SDS-PAGE bestimmt werden.

10. Ein Verfahren zum Herstellen eines Phagenklons λgt11, enthaltend ein Insert, das für einen Teil eines Oberflächenproteins von Mycoplasma hyopneumoniae kodiert, ausgewählt aus der Gruppe bestehend aus Protein A, Protein C, Protein D und Protein E, worin ein 0,5 kb-Insert, das für einen Teil des Proteins E kodiert, in die EcoRI-Stelle von λgt11 eingefügt wird, wobei das Protein E ein Molekulargewicht von 43 kD besitzt, wobei die Molekulargewichte der Proteine durch SDS-PAGE bestimmt werden.

## Revendications (Revendications pour l'(les) Etat(s) contractant(s) suivant(s): BE, CH, LI, DE, FR, GB, IT, LU, NL, SE)

1. Protéine de surface de *Mycoplasma hyopneumoniae,* choisie parmi la protéine A, la protéine C, la protéine D et la protéine E,
lesdites protéines étant reconnues par au moins l'une des sources suivantes d'anticorps: sérum porcin immun, sérum de lapin anti-mycoplasme hyper-immun et anticorps monoclonaux anti-mycoplasme de souris, et
ladite protéine A ayant une masse moléculaire de 105 kDa,
ladite protéine C ayant une masse moléculaire de 85 kDa,
ladite protéine D ayant une masse moléculaire de 70 kDa, étant entendu que la protéine p68 selon EP-A-0 196 215 est exclue, et
ladite protéine E ayant une masse moléculaire de 43 kDa, les masses moléculaires des protéines étant déterminées selon SDS-PAGE.

2. Clone de phage λgt11 contenant un insert de 1,5 kb dans le site *Eco*RI de λgt11, lequel insert code pour une partie de la protéine A de la revendication 1.

3. Clone de phage λgt11 contenant un insert de 0,5 kb dans le site *Eco*RI de λgt11, lequel insert code pour une partie de la protéine C de la revendication 1.

4. Clone de phage λgt11 contenant un insert de 3,2 kb dans le site *Eco*RI de λgt11, lequel insert code pour une partie de la protéine D de la revendication 1.

5. Clone de phage λgt11 contenant un insert de 0,5 kb dans le site *Eco*RI de λgt11, lequel insert code pour une partie de la protéine E de la revendication 1.

6. Procédé d'ADN recombinant pour la production des protéines de la revendication 1, qui sont capables d'induire une réponse antigénique, comprenant:
(a) la préparation d'une séquence d'ADN capable d'induire un micro-organisme hôte à produire la protéine;
(b) le clonage de la séquence d'ADN dans un vecteur capable d'être transféré et de se répliquer dans un micro-organisme hôte, ce vecteur contenant des éléments fonctionnels pour la séquence d'ADN;
(c) le transfert du vecteur contenant la séquence d'ADN et les éléments fonctionnels dans un micro-organisme hôte capable d'exprimer la protéine antigénique;
(d) la culture du micro-organisme hôte dans des conditions appropriées à l'amplification du vecteur et à l'expression de la protéine; et
(e) dans cet ordre ou l'autre:
I) la récolte de la protéine; et
II) la contrainte de la protéine à adopter une structure lui conférant des propriétés antigéniques analogues aux propriétés présentées par des mycoplasmes.

## Revendications (Revendications pour l'(les) Etat(s) contractant(s) suivant(s): AT)

1. Protéine de surface de *Mycoplasma hyopneumoniae,* choisie parmi la protéine A, la protéine C, la protéine D et la protéine E,
lesdites protéines étant reconnues par au moins l'une des sources suivantes d'anticorps: sérum porcin immun, sérum de lapin anti-mycoplasme hyper-immun et anticorps monoclonaux anti-mycoplasme de souris, et
ladite protéine A ayant une masse moléculaire de 105 kDa,
ladite protéine C ayant une masse moléculaire de 85 kDa,
ladite protéine D ayant une masse moléculaire de 70 kDa,
étant entendu que la protéine p68 selon EP-A-0 196 215 est exclue, et
ladite protéine E ayant une masse moléculaire de 43 kDa, les masses moléculaires des protéines étant déterminées selon SDS-PAGE.

2. Clone de phage λgt11 contenant un insert de 1,5 kb dans le site *Eco*RI de λgt11, lequel insert code pour une partie de la protéine A de la revendication 1.

3. Clone de phage λgt11 contenant un insert de 0,5 kb dans le site *Eco*RI de λgt11, lequel insert code pour une partie de la protéine C de la revendication 1.

4. Clone de phage λgt11 contenant un insert de 3,2 kb dans le site *Eco*RI de λgt11, lequel insert code pour une partie de la protéine D de la revendication 1.

5. Clone de phage λgt11 contenant un insert de 0,5 kb dans le site *Eco*RI de λgt11, lequel insert code pour une partie de la protéine E de la revendication 1.

6. Procédé d'ADN recombinant pour la production des protéines de la revendication 1, qui sont capables d'induire une réponse antigénique, comprenant:
(a) la préparation d'une séquence d'ADN capable d'induire un micro-organisme hôte à produire la protéine;
(b) le clonage de la séquence d'ADN dans un vecteur capable d'être transféré et de se répliquer dans un micro-organisme hôte, ce vecteur contenant des éléments fonctionnels pour la séquence d'ADN;
(c) le transfert du vecteur contenant la séquence d'ADN et les éléments fonctionnels dans un micro-organisme hôte capable d'exprimer la protéine antigénique;
(d) la culture du micro-organisme hôte dans des conditions appropriées à l'amplification du vecteur et à l'expression de la protéine; et
(e) dans cet ordre ou l'autre:
I) la récolte de la protéine; et
II) la contrainte de la protéine à adopter une structure lui conférant des propriétés antigéniques analogues aux propriétés présentées par des mycoplasmes.

7. Procédé pour la production d'un clone de phage λgt11 contenant un insert codant pour une partie d'une protéine de surface de *Mycoplasma hyopneumoniae,* choisie parmi la protéine A, la protéine C, la protéine D et la protéine E, dans lequel un insert de 1,5 kb, codant pour une partie de la protéine A, est inséré dans le site *EcoRI* de λgt11, ladite protéine A ayant une masse moléculaire de 105 kDa, les masses moléculaires des protéines étant déterminées selon SDS-PAGE.

8. Procédé pour la production d'un clone de phage λgt11 contenant un insert codant pour une partie d'une protéine de surface de *Mycoplasma hyopneumoniae,* choisie parmi la protéine A, la protéine C, la protéine D et la protéine E, dans lequel un insert de 0,5 kb, codant pour une partie de la protéine C, est inséré dans le site *EcoRI* de λgt11, ladite protéine C ayant une masse moléculaire de 85 kDa, les masses moléculaires des protéines étant déterminées selon SDS-PAGE.

9. Procédé pour la production d'un clone de phage λgt11 contenant un insert codant pour une partie d'une protéine de surface de *Mycoplasma hyopneumoniae,* choisie parmi la protéine A, la protéine C, la protéine D et la protéine E, dans lequel un insert de 3,2 kb, codant pour une partie de la protéine D, est inséré dans le site *EcoRI* de λgt11, ladite protéine D ayant une masse moléculaire de 70 kDa, les masses moléculaires des protéines étant déterminées selon SDS-PAGE.

10. Procédé pour la production d'un clone de phage λgt11 contenant un insert codant pour une partie d'une protéine de surface de *Mycoplasma hyopneumoniae,* choisie parmi la protéine A, la protéine C, la protéine D et la protéine E, dans lequel un insert de 0,5 kb, codant pour une partie de la protéine E, est inséré dans le site *EcoRI* de λgt11, ladite protéine E ayant une masse moléculaire de 43 kDa, les masses moléculaires des protéines étant déterminées selon SDS-PAGE.
